# EUROPEAN PATENT SPECIFICATION

(11) **EP 3 294 393 B1**
(45) Date of publication and mention of the grant of the patent: **25.03.2020**
(21) Application number: 16722527.5
(22) Date of filing: 12.05.2016
(51) Int. Cl.: A61M 15/06, A24F 47/00

(54) **NICOTINE DELIVERY DEVICE**
NIKOTINFREISETZUNGSVORRICHTUNG
DISPOSITIF D'ADMINISTRATION DE NICOTINE

(30) Priority: 13.05.2015 EP 15167708
(43) Date of publication of application: 21.03.2018
(73) Proprietor: Smpl Innovations Ltd., Brighton, BN1 1UT (GB)
(72) Inventor: MOALLEMI, Omid, Hassocks Sussex BN6 8NT (GB); MATHIAS, Michael, 8400 Winterthur (CH); PARR, David, Brighton BN1 4EW (GB); EINHÄUSER, Daniel, 22085 Hamburg (DE)
(74) Representative: Müller Schupfner & Partner Patent- und Rechtsanwaltspartnerschaft mbB
(86) International application number: PCT/EP2016/000773
(87) International publication number: WO 2016/180534

(56) References cited:
- EP-A1- 2 399 637
- WO-A1-98/44961
- WO-A2-91/19524
- FR-A1- 2 654 002
- GB-A- 2 466 758
- US-A- 3 884 246
- US-A- 5 865 186

## Description

The present invention refers to a nicotine delivering device in the form of a smokeless cigarette. The smokeless cigarette device according to the present invention incorporates at least one capsule. The at least one capsule comprises at least one chamber having an opening at both of its ends, each of which is closed by a lid that both open simultaneously upon pressure being applied to the outside of the capsule.

### BACKGROUND OF THE INVENTION

Smokeless cigarettes releasing nicotine are known in the art. Since a number of years there is an emerging market for products that deliver nicotine without producing a passive smoking effect. Several products have been suggested some of which involve heat or vapor generation or batteries.

Smokeless cigarettes were suggested comprising capsules that break when pressure is applied from outside thereby releasing the ingredients kept inside the capsule. One Example is EP 0192950 A1 which discloses a capsule comprising a predetermined breaking point opening the capsule upon pressure applied from outside. According to a further embodiment the capsule comprises a valve opening upon sucking on the smokeless cigarette. US 3347231 is a further example disclosing an "imitation cigarette" with nicotine in a liquid state and optionally flavouring ingredients contained in an ampule. The ampule is disposed in the cigarette generally centrally and comprises a weakened section that easily breaks upon outside pressure. Upon breakage the emerging liquid is absorbed into the surrounding filter materials for an extended release of the ingredients. Yet another similar embodiment with a frangible capsule is disclosed in WO 98/44961 A1.

EP 2399637 teaches a non-heated smokeless cigarette comprising a container open at either end and a filler arranged in the container. Air-permeable caps are fitted to either end of the container.

Numerous other designs have been proposed for smokeless cigarettes such as an electronic stimulated cigarette having a reservoir in the form of a capsule, a heater and a spray device (GB 2466758 A), a smokeless cigarette having a deformable cylinder as a reservoir having valves (US 5865186 A), smokeless cigarettes comprising an impervious casing with a plug out of a porous filter material (US 3884246 A) or with a temporarily re-lockable container (FR 2654002 A1). WO 98/44961 A1 discloses an odorizer or freshener having a rod like shape that resamples the shape of a conventional cigarette in which according to one embodiment a fragrance is embedded in a breakable fragrance-containing capsule.

### OBJECT OF THE PRESENT INVENTION

It is the object of the present invention to provide a one-use, disposable device that can have the look and the outside of a conventional cigarette but neither is in need for a combustion process generating fume, an electric heating device, electronics or a battery. Further long lasting and uniform nicotine and/or flavour delivery is wanted as well as easy handling and a pleasant consumption.

### SUMMARY OF INVENTION

The subject matter of the present invention is described in the independent claims. Preferred embodiments are disclosed in the sub-claims or are described herein under.

The smokeless cigarette device of the present invention typically emulates the look of a conventional cigarette and comprises at least one capsule, preferably one capsule. The capsule comprises one or more chambers, each chamber for storing one or more ingredients. The chamber has the form of a tube having a first end and a second end. At each end there is placed a removable lid. Each chamber is closed by the two lids, one at each end. If the capsule comprises more than one chamber, each chamber may comprise its own lid at each end. In another embodiment one lid may close more than one opening at each end or the lid consists of two or more interconnected lids.

The capsule is flexible upon pressure being applied from outside. The user compresses the capsule/chamber(s) by pressing preferably on the outer surface of the device.

By this action the lids at each end are removed from the ends of the chamber. Upon removal of the lids, the contents of the chamber(s) exits the chamber(s) / capsule preferably into the surrounding filter which allows the air to mix with it before entering the users mouth. The chamber(s) are preferably reversible deformable upon the user applying a pressure on the outer surface of the smokeless cigarette device. When the consumer draws air through the device, the ingredient in the tube will be drawn out and taken up by air. If more than one ingredient is used, the ingredients are mixed at least in the air stream, typically, however, already in the absorbent used to soak up the ingredients.

The ingredients contained in the chamber are for example nicotine, nicotine derivatives, steam generating chemicals, heat generating chemicals, flavours or mixtures thereof, preferably each contained in a separate chamber. Typically, nicotine and/or nicotine derivatives are contained in one chamber and a flavour is comprised separately in a second chamber of the capsule. The two chambers form a two compartment capsule, having adjacent chambers side by side. For this purpose, the capsule preferably comprises at least two chambers, linked with each other preferably along their longitudinal side.

The ingredient are typically liquids preferably a solution of one or more ingredients dissolved in a solvent. However, it is also possible to use ingredients in solid form, e.g. as a powder or to store a solvent and the solid ingredient in different chambers and to dissolve the ingredient in the solvent only when the capsule opens and the contents of the chambers are released and mixed. Preferably at least one ingredient is a liquid. Typical ingredients are nicotine, nicotine derivatives and/or flavours.

### DETAILED DESCRIPTION OF THE INVENTION

The present smokeless cigarette device is a nicotine delivery device that delivers one or more ingredients such as nicotine and optionally flavour to its user without the aid of combustion or batteries. It achieves this by using a capsule containing in its inside ingredients such as nicotine and optionally further ingredients such as flavours, solvents or chemical heat generators or fume generators. The ingredients (including the nicotine) are released by the parallel opening of the lids at each end of each chamber.

The lids open upon the application of a pressure by the user on the outer surface of the device in the region where the capsule is located. This action allows the release of the ingredients, their uptake by the air flowing through the device, in particular the chamber(s), and their mixing at least in the air when more than one ingredient is used.

Preferred is a capsule with a double chamber that separates nicotine and flavour, preferably both in liquid form. If the nicotine and flavour are mixed together in liquid form prior to use, the different ingredients can make unwanted chemical reactions and degrade over time which will have a negative impact on the performance and taste. This can, for instance, lead to poor nicotine delivery and flavour quality over time. By separating the ingredients until activation, it guarantees the best possible experience for the user in that the ingredients always freshly interact only after they are released by the user. The chambers are resistant to the ingredients contained therein, so that they can be stored therein without degeneration.

The lids are attached and held to the capsule preferably based on a frictional fit in particular based on a frictional fit of the outer diameter of the lid against the inner diameter of the chamber.

According to one embodiment, the lid has a slightly larger circumference than the circumference of the chamber at the first and second end. If the lids are pushed into the chamber, the chamber expands at the first and second end and the outer circumferential surface area of the lids are in a frictional interaction with the inner circumference of the two ends of the chambers). Due to the elastic enlargement of the circumference of the two ends of the chamber(s) the outer ends firmly press against the lid. This results in a gas and fluid tight mechanical fit without any securing aid such as a glue.

To release the lid from the capsule and to allow the ingredients to exit, the user applies pressure to the external area of the device, preferably at one or two points that are located approximately opposite to each other that are preferably indicated by one or more indicator, e.g. two visual dots, on the printed outer side of the overwrap. The pressure applied by the user's fingers impacts on the capsule and the chamber walls are compressed.

Upon application of such force, the chamber resiliently bends and the tube will taper with a change of profile in circumference towards the ends. This will push out the lids. The lids will be forced at both ends to move away from the capsule body. The force applied will alter the shape of the capsule/chamber(s) into an oval shape. This change of shape promotes the removal of the lids and helps to move the lids away by applying force along the lids' bottom radius. The tapered form of the chamber and the interaction of the chamber wall with the bottom radius of the lid helps to push the lid out of the chamber against the friction that holds it in place.

According to one embodiment the lids have a tubular form. Each lid is preferably further characterized in that the bottom circumference of the lid is smaller than the head and/or middle circumference. The lid may be hollow, particular at the inside end.

According to this embodiment the chamber may also have a tubular form which is tapered towards the middle section with smaller circumference in the middle as at the end. The main longitudinal axis of the capsule/chamber(s) is/are orientated in parallel to the main axis of the device. According to one preferred embodiment the outer circumference of the capsule leans against the internal wall of the capsule holder along at least two opposite lines of the outer dimension of the capsule. The opposite lines are parallel to the main axis of the device. As a result the capsule is mounted in the centre of the capsule with respect to a sectional view rectangular to the main axis of the device.

Once the lids are released, they have enough room to travel away from the capsule in order not to block the entrance of the chambers and to allow airflow to pass through the chamber and to draw the liquid out into the surrounding filters. The lids are smaller in size than the empty cavity extending at each end of the capsule. The capsule can therefore freely move away from each end of the chamber.

The chambers are preferably made out of a polymer material that is resilient (e.g. elastic compressible), so that if at least one chamber is compressed in a middle region of its longitudinal dimension both lids exit the chamber so that the chamber is opened at both ends preferably simultaneously.

According to one embodiment to activate the product, the user applies force at a place given by the one or two indicators on the outer body overwrap. This force (typically 5N ± 2.5N (250kPa±125kPA) based on a load area with a 5 mm diameter) is transferred to the capsule which is compressed and with this, the capsule body is deformed. The lids are held to the capsule body by a frictional fit, but when the capsule body deforms, it alters the shape of the capsule body and the interference fit is then removed from the design allowing the lids to become loose.

Pressure (hydraulic and/or pneumatic) inside the chamber(s) created by the user further supports the pushing out of the lids away from the capsule and into the cavity of the capsule holder. According to one embodiment the capsule/chamber(s) are filed by the ingredients to at least 80 vol.%, preferably at least 90 vol.%, so that upon compression of the capsule/chamber(s) a hydraulic pressure is generated that removes the lids or helps to remove the lids.

Because the capsule is shorter in length than the cavity within the capsule holder there is according to one embodiment around 2 to 6 mm, preferably about 3 mm, at each end of the capsule filter an empty space. This empty part of the cavity is for the lids to move into after being removed from the chamber. If the capsule would be too close to the body filter and mouth filters, the lids would not be able to move into an empty space, possibly resulting in the liquids staying at least partially inside the capsule or an insufficient airflow through the chambers to take up the ingredients released.

Once the capsule/chamber(s) has/have been indented and the lids at each end have moved out of the way, the capsule is open and there is a clear air path that flows through the product allowing air drawn through to pass around and through the capsule body, drawing the ingredient(s) out and into the surrounding filters.

The user then draws air through the product which allows air to enter at the other end of the inlet body.

By this time, the liquid has started to escape the capsule, it flows into the surrounding areas, mainly, the capsule holder and absorbent material covering the inside of the cavity. Any residual liquid still inside the capsule will be drawn out and into the absorbent material as the user draws the air through it.

At this stage, the ingredients, such as the nicotine and the flavour solutions, are mixing in the filter and air flow is slowly wicking them together. This mixture of air and liquid is then drawn through the mouth filter and into the user's mouth where it hits the tongue for the perception of its flavour and/or the nicotine.

The device according to one preferred embodiment has a tubular form, having a length of approximately 60 to 120 mm, preferably 80 to 90 mm, in particular about 85 mm long and a diameter of 4 to 16 mm, preferably 6 to 9 mm, in particular about 7.5 mm.

The smokeless cigarette device comprises a mouth filter, the capsule, a capsule holder, an inlet body and an overwrap. According to one embodiment the capsule holder and the inlet body form one integral body and according to another embodiment the mouth filter and the capsule holder form one integral body and according to another preferred embodiment all component form separate but interconnectable bodies.

Suitable materials are for example mono acetate filters made from cellulose acetate fibres, carbon based filters, natural fibre filters derived from cotton or Ochre filters. The filter material may be biodegradable.

The first component is the mouth filter and is situated at the beginning of the device, i.e. close to the mouth when the device is in use. Amongst others the purpose of the mouth filter is to provide structure to the device and to better mix the air comprising the ingredients, as well as to stop the ingredients to enter into the user's mouth directly without being diluted by air. The mouth filter is typically 5 to 20 mm in length, preferably about 10 mm.

As the user sucks on the product, air is drawn through the inlet body, through the capsule holder, through the capsule and finally through the mouth filter before entering the user's mouth. Along the way, the flow of air draws out any residual ingredients that are still inside the capsule and according to one embodiment that are released into an absorbent.

According to one embodiment the mouth filter is made out of a closed cell sponge material. This material can preferably be made from foamed polyolefins, e.g. polyethylene, or foamed polyurethane, with a pore size of 1 to 4 mm, preferably around 0.3 mm, which is believed to aid the creation of fine droplets ready to enter the users mouth.

Further advancing from the mouth filter towards the second end there is located a capsule holder. The capsule holder may be made out of the same material as the mouth filter or the inlet body and is in the form of a hollow tube. The wall thickness is preferably 1 to 5 mm, preferably about 2.5 mm. The capsule holder acts as structural element and contains the capsule in its hollow middle.

When the ingredients are released from the capsule, they will come into contact with the inner wall of the capsule holder. As the user draws air though the product, the ingredients move in the same direction as the flow of air when they exit the chamber(s) and will get into contact with the inner circumferential surface of the capsule holder and will disperse into the wall of the capsule holder. This helps the ingredients due to a delayed release out of the wall to deliver a steady and slow release of nicotine and flavour into the air stream and finally into the user's mouth.

Next in the design of the smokeless cigarette device is the inlet body which typically is the longest component in the assembly. It is providing structure and integrity to the design.

An overwrap is applied to all the components during assembly so that it holds them all together, forming the smokeless cigarette shape. This is achieved using automated machinery, which in principle is the same as that used in existing cigarette manufacture machines. The overwrap can be made from standard cigarette paper such as hemp, rice straw or flax mixed with wood pulp. A waterproof coating or glue will be preferably applied on the inside surface of the paper which will not allow liquid to penetrate through so that the liquid nicotine and flavour solutions cannot reach the outer surface of the product.

The overwrap preferably comprises as water proof membrane on the inside surface to keep liquids from penetrating to the outer surface. The water proof membrane can be made from a hydrophobic coating or a plastic material. The outer material may be amongst others paper or card.

This outer wrap paper of the overwrap can also be flavoured, so that it has a desired taste for the user to experience whilst sucking on the device. For example, a peppermint flavour will enhance the sensation of flavour in tandem with the internal flavour inside the capsule(s).

A trade name, design and logo can be applied to this overwrap paper during the assembly manufacture process as wanted. The target thickness for this paper is preferably about 0.12 mm. The waterproof coating will be applied to the paper during the manufacture process if the paper which gives the paper the properties of a non-permeable membrane.

Once all components are in place after assembly, the product will deliver a specified amount of liquid and nicotine to the user for up to 1-2 hours and at least 10 draws, ideally 20-50.

Oral absorption of nicotine is bitter, so by masking this taste using flavours adds value to the product by giving the user a better experience. Flavours to use as ingredient can be Peppermint, Apple, Mint, Vanilla, Raspberry, American Tobacco Blend or Virginia Tobacco Blend to name but a few.

Another possible feature is that there is a reaction after activation to indicate to the user that the product has come to the end of its use and that the user should not use it any more. This can be achieved by a chemical reagent that activates the filter or overwrap to show a visual indication that the product is now useless, e.g. by reaction with saliva or one of the ingredients. For example, the saliva can start off a chemical reaction on the mouth filter that slowly let the mouth filter become a different colour after roughly 2 hours, similar to a pregnancy test reaction or visual indicators that are present on baby nappies to show that the baby has wet him or herself.

The product is intended to have taggants, which is a chemical/physical marker that allows a code to be applied to a product batch, to help stop counterfeiting.

### DESCRIPTION OF THE DRAWINGS

The invention is further illustrated by the drawings without being limited thereto.

The figures show:
- Fig.1: a view of the nicotine delivering device with the second end, where the air is drawn into the product, in the foreground;
- Fig. 2: an exploded view with the overwrap, the mouth filter and the capsule removed;
- Fig.3: a sectional view of the product, with the capsule holder partially opened;
- Fig.4: a double with two chambers inside the capsule holder with the lids removed;
- Fig.5: a double capsule with the two interconnected lids removed;
- Fig.6: a three dimensional view of the capsule inside the device with the lids removed and air passing through the device;
- Fig.7: a sectional view depicting a double capsule and how it is located in the capsule holder;
- Fig.8: a closer view of one lid and one end of a chamber; and
- Fig.9: a view of one chamber with the chamber walls inclined due to outside pressure being applied and the lids exiting the chambers.

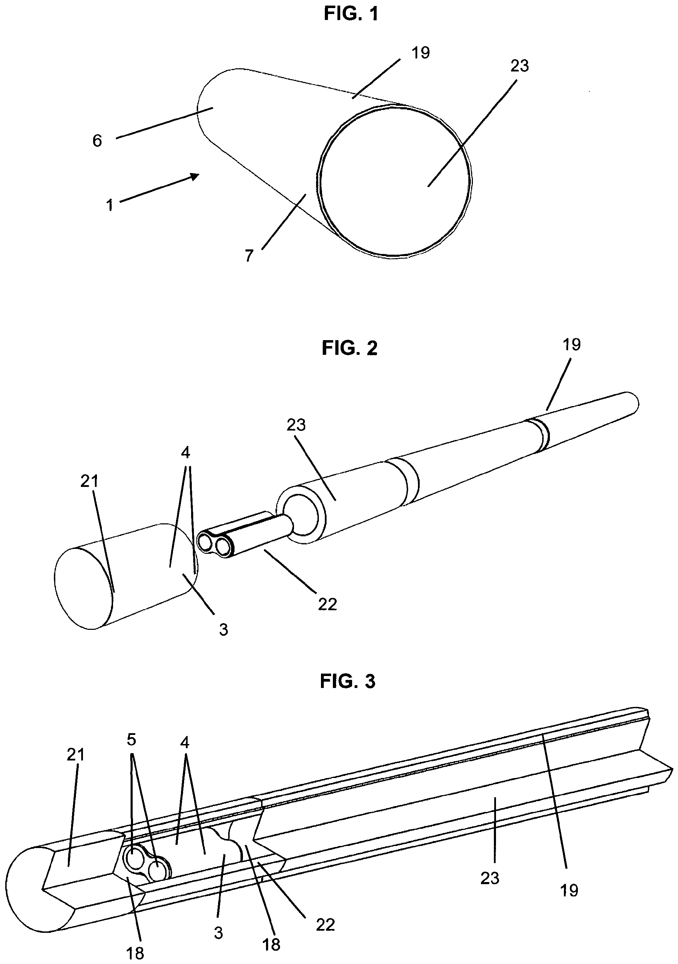

Fig. 1 shows the outside of the present smokeless cigarette device 1 viewed from the second end 7 towards the first end 6 with the mouth filter 21. In the front there is the inlet body 23 covered by the overwrap 19. The mouth filter 21 is not visible, because it is covered by the overwrap 19.

Fig. 2 depicts the device in an exploded view showing the mouth filter 21, being a solid (possibly porous) body, the hollow capsule holder 22 and the inlet body 23.

Mounted inside the cavity of the capsule holder 22 is a capsule 3 with two chambers 4. One chamber 4 is filled with nicotine the other with a flavour.

The placement of the capsule 3 in the cavity of the capsule holder 22 is better visible in Fig. 3 where due to a sectional opening of the capsule holder the empty space 18 in front of and behind the capsule is shown. The empty space 18 takes up the lids when released. Each chamber 4 comprises a lid 5 at each end. The two lids 5 at one end are interconnected. The lids 5 are attached and held to each chamber wall based on a frictional fit of the outer diameter of the lid against the inner diameter of the chamber.

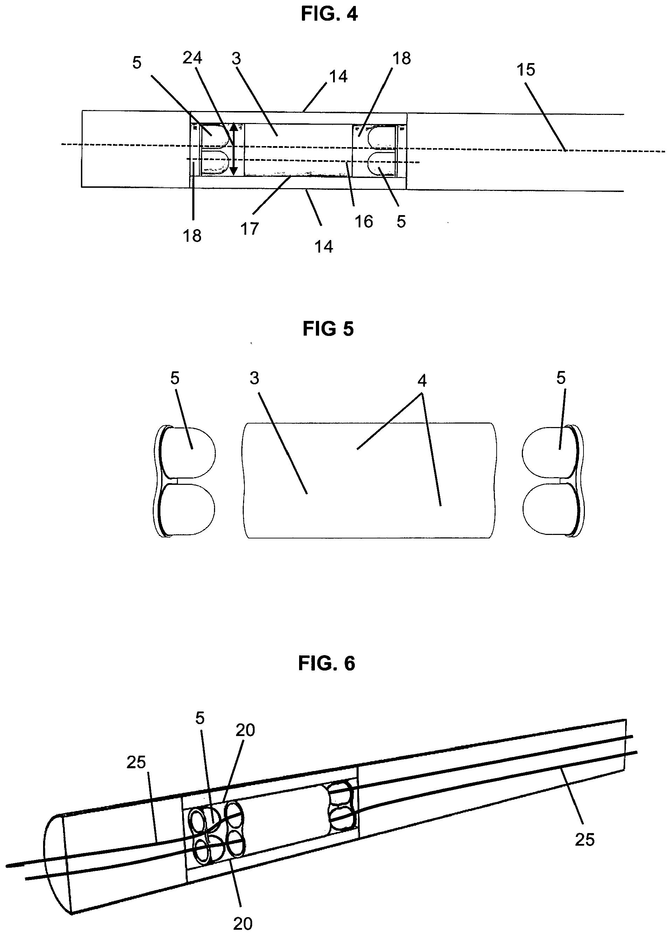

Fig. 4 shows that the main longitudinal axis 16 of each of the chambers is parallel to the main axis of the device. The interconnected lids 5 are removed.

Fig. 5 depicts the capsule 3 separate from the rest of the device. Both chambers 4 are interconnected and form an oval at the outside as well as both lids 5 at one end.

The flow of air 25 through the opened chambers is depicted in Fig.6. The adsorbent 20 acts as a retardant allowing a steady and delayed delivery of the ingredients into the flow of air.

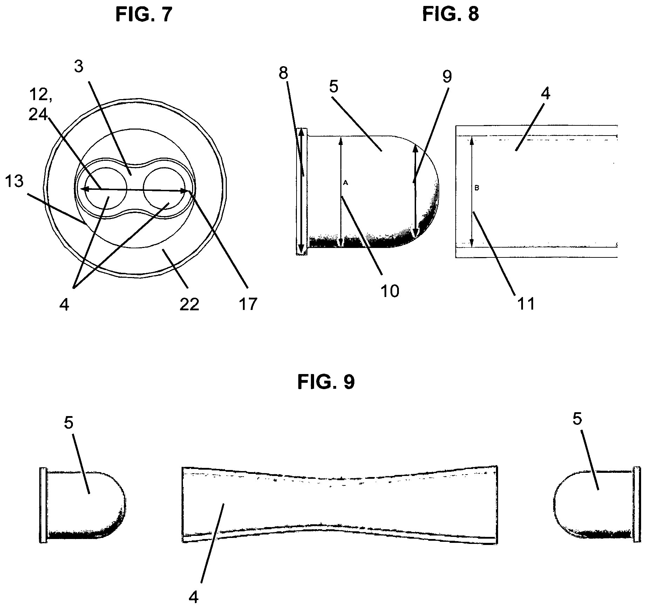

Each lid has a slightly larger diameter 10 at the head than the inner diameter 9 of the chamber 11 at the first and second end (Fig.7). In the pushed-in state of the lid the chamber is slightly expanded at its first and second end. Due to the elastic enlargement of the circumference of the ends of the chambers the outer ends firmly press against the corresponding lid 5.

To release the lid from the capsule and to allow the ingredients to exit, the user applies pressure to the external area of the device. The pressure applied by the user's fingers impacts on the capsule and the chamber walls are compressed and the tube(s) taper. The lids 5 are forced at both ends to move away from the capsule body. The tapered form of the chamber and the interaction of the chamber wall with the narrower bottom radius 9 of the lid 5 pushes the lid 5 out of the chamber against the friction that held it in place.

The lids 5 have a tubular form with the bottom circumference of the lid being smaller than the head circumference. The lid 5 is hollow at the outside end (= head end (11)) and round at the inside end (= bottom end (9)) with a cupola form.

Once the lids 5 are released, they have enough room 18 available to travel away from the capsule in order not to block the entrance of the chambers and to allow airflow to pass through the chamber and to draw the liquid out into the surrounding filters. The lids are smaller in size than the empty cavity 18 extending at each end of the capsule. The capsule can therefore freely move away from each end of the chamber.

Once the capsule 3 /chamber(s) 4 has been indented and the lids 5 have moved out of the way, the chamber 4 is open and there is a clear air path 25 that flows through the product allowing air drawn through to pass around and through the capsule body, drawing the ingredient(s) out and into the surrounding filters.

The user then draws air through the product which allows air to enter at the other end of the inlet body.

**LIST OF REFERENCE NUMERALS**

| | |
|---|---|
| smokeless cigarette / nicotine delivery device | 1 |
| ingredients (nicotine and optionally flavours) | 2 |
| capsule | 3 |
| chamber | 4 |
| lid(s) | 5 |
| first end of the device | 6 |
| second end of the device | 7 |
| outer diameter of the lid | 8 |
| bottom diameter of the lid | 9 |
| head diameter of the lid | 10 |
| inner diameter of the chamber | 11 |
| outer circumference of the capsule | 12 |
| internal wall of the capsule holder | 13 |
| spots opposite to each other / visual dots | 14 |
| main axis of the device | 15 |
| main longitudinal axis of the chamber | 16 |
| lines of contact of the capsule with the capsule holder | 17 |
| empty space in the cavity | 18 |
| overwrap | 19 |
| absorbent (for the ingredients) | 20 |
| mouth filter | 21 |
| capsule holder | 22 |
| inlet body | 23 |
| inner diameter of the capsule holder | 24 |
| stream of air | 25 |

## Claims

1. Smokeless cigarette device (1) comprising one or more capsules (3), each capsule (3) comprising at least one chamber (4) having an opening at each of its ends with the openings at opposite ends of the chamber, wherein each of the two openings is closed by a lid (5), wherein the lids (5) at opposite ends of the chamber (4) open upon pressure being applied to the outside of the capsule (3).

2. Device according to claim 1 comprising one capsule (3) having two or more chambers (4), each filled with a different ingredient (2), wherein preferably at least one ingredient (2) is nicotine or a nicotine derivative.

3. Device according to claim 1 or 2, wherein one lid (5) closes more than one opening at each end or the lids (5) at one end consist of two or more interconnected lids (5).

4. Device according to one or more of the preceding claims, wherein the at least one chamber (4) has a tubular form and the capsule (3) and the chamber (4) is flexible when pressure is applied from outside.

5. Device according to one or more of the preceding claims, wherein the ingredients (2) are selected from one or more members of the group nicotine, nicotine derivatives, steam generating chemicals, heat generating chemicals and flavours.

6. Device according to one or more of the preceding claims, wherein the ingredients (2) are in a liquid or dissolved state, preferably a solution of one or more ingredients dissolved in a solvent.

7. Device according to one or more of the preceding claims, wherein the lids (5) are attached and held to the capsule (3) solely based on a frictional fit, in particular a fit of the outer circumference of the lid against the inner circumference of the chamber.

8. Device according to one or more of the preceding claims, wherein the lid (5) has a slightly larger outer circumference than the inner circumference of the chamber (4) at the end where the opening is.

9. Device according to one or more of the preceding claims, wherein the lids (5) have a frustoconical type of shape with a smaller diameter at the bottom end (9) of the lid and a larger diameter at the head end (11) or where the bottom circumference of the lid is smaller than the head circumference.

10. Device according to one or more of the preceding claims, wherein the chamber(s) (4) have a tubular form wherein the chamber (4) preferably tapers with an increase in circumference towards each end and a smaller circumference in the middle.

11. Device according to one or more of the preceding claims, wherein the capsule and the chambers are made out of a polymer material that is resilient, so that the chamber walls are elastically compressible by a user's fingers impact on the capsule.

12. Device according to one or more of the preceding claims, wherein the device comprises a mouth filter (21), an inlet body (23) and a capsule holder (22) in between the mouth filter (21) and the inlet body (23), wherein the capsule holder (22) has a wall surrounding a cavity and the cavity comprises the capsule (3).

13. Device according to claim 12, wherein the device comprises an absorbent material inside the cavity, preferably wherein the absorbent material soaks up the ingredient and the ingredient travels through the wall of the cavity to be uptaken by the air flowing at the outer surfaces.

14. Device according to one or more of the preceding claims wherein the cavity comprises empty spaces (18) at each end of the capsule having a size that allows the lids to move away from the chamber when removed from the chamber.

15. Device according to one or more of the preceding claims, wherein an overwrap (19) is provided covering at least the inlet body (23) and the capsule holder (22) and optionally also the mouth filter (21).

## Patentansprüche

1. Rauchlose Zigarettenvorrichtung (1), umfassend eine oder mehrere Kapseln (3), wobei jede Kapsel (3) mindestens eine Kammer (4) umfasst, die an jedem ihrer Enden eine Öffnung aufweist, wobei sich die Öffnungen an entgegengesetzten Enden der Kammer befinden, wobei jede der beiden Öffnungen mit einer Kappe (5) verschlossen ist, wobei sich die Kappen (5) an entgegengesetzten Enden der Kammer (4) nach dem Ausüben von Druck auf die Außenseite der Kapsel (3) öffnen.

2. Vorrichtung nach Anspruch 1, umfassend eine Kapsel (3) mit zwei oder mehreren Kammern (4), jede gefüllt mit einem anderen Inhaltsstoff (2), wobei vorzugsweise mindestens ein Inhaltsstoff (2) Nicotin oder ein Nicotinderivat ist.

3. Vorrichtung nach Anspruch 1 oder 2, wobei eine Kappe (5) mehr als eine Öffnung an jedem Ende verschließt oder die Kappen (5) an einem Ende aus zwei oder mehreren miteinander verbundenen Kappen (5) bestehen.

4. Vorrichtung nach einem oder mehreren der vorangehenden Ansprüche, wobei die mindestens eine Kammer (4) eine röhrenförmige Form aufweist und die Kapsel (3) und die Kammer (4) flexibel sind, wenn Druck von außen ausgeübt wird.

5. Vorrichtung nach einem oder mehreren der vorangehenden Ansprüche, wobei die Inhaltsstoffe (2) ausgewählt sind aus einem oder mehreren Vertretern der Gruppe Nicotin, Nicotinderivate, dampferzeugende Chemikalien, wärmeerzeugende Chemikalien und Aromen.

6. Vorrichtung nach einem oder mehreren der vorangehenden Ansprüche, wobei die Inhaltsstoffe (2) in flüssigem oder gelöstem Zustand vorliegen, vorzugsweise eine Lösung von einem oder mehreren Bestandteilen, gelöst in einem Lösungsmittel, sind.

7. Vorrichtung nach einem oder mehreren der vorangehenden Ansprüche, wobei die Kappen (5) ausschließlich basierend auf einer Reibungspassung, insbesondere einer Passung des äußeren Umfangs der Kappe gegen den inneren Umfang der Kammer, an der Kapsel (3) angebracht sind und gehalten werden.

8. Vorrichtung nach einem oder mehreren der vorangehenden Ansprüche, wobei die Kappe (5) einen äußeren Umfang aufweist, der etwas größer als der innere Umfang der Kammer (4) an dem Ende, wo sich die Öffnung befindet, ist.

9. Vorrichtung nach einem oder mehreren der vorangehenden Ansprüche, wobei die Kappen (5) eine kegelstumpfartige Form mit einem kleineren Durchmesser an dem unteren Ende (9) der Kappe und einem größeren Durchmesser an dem Kopfende (11) aufweisen oder wobei der untere Umfang der Kappe kleiner als der Kopfumfang ist.

10. Vorrichtung nach einem oder mehreren der vorangehenden Ansprüche, wobei die Kammer(n) (4) eine röhrenförmige Form aufweist/aufweisen, wobei die Kammer (4) vorzugsweise mit einer Zunahme des Umfangs in Richtung jedes Endes und einem kleineren Umfang in der Mitte konisch zuläuft.

11. Vorrichtung nach einem oder mehreren der vorangehenden Ansprüche, wobei die Kapsel und die Kammern aus einem Polymermaterial, das elastisch ist, hergestellt sind, sodass die Kammerwandungen durch eine Einwirkung der Finger des Anwenders auf die Kapsel elastisch zusammendrückbar sind.

12. Vorrichtung nach einem oder mehreren der vorangehenden Ansprüche, wobei die Vorrichtung einen Mundfilter (21), einen Einlasskörper (23) und eine Kapselhalterung (22) zwischen dem Mundfilter (21) und dem Einlasskörper (23) umfasst, wobei die Kapselhalterung (22) eine Wandung, die einen Hohlraum umgibt, aufweist und der Hohlraum die Kapsel (3) umfasst.

13. Vorrichtung nach Anspruch 12, wobei die Vorrichtung ein absorbierendes Material in dem Hohlraum umfasst, vorzugsweise wobei das absorbierende Material den Inhaltsstoff aufsaugt und der Inhaltsstoff die Wandung des Hohlraums passiert, um durch die an den Außenflächen strömende Luft aufgenommen zu werden.

14. Vorrichtung nach einem oder mehreren der vorangehenden Ansprüche, wobei der Hohlraum an jedem Ende der Kapsel Leerräume (18) umfasst, die eine Größe aufweisen, die das Wegbewegen der Kappen von der Kammer beim Lösen von der Kammer ermöglicht.

15. Vorrichtung nach einem oder mehreren der vorangehenden Ansprüche, wobei eine Umhüllung (19) vorgesehen ist, die zumindest den Einlasskörper (23) und die Kapselhalterung (22) und gegebenenfalls auch den Mundfilter (21) umhüllt.

## Revendications

1. Dispositif de cigarette sans fumée (1) comprenant une ou plusieurs capsules (3), chaque capsule (3) comprenant au moins une chambre (4) ayant une ouverture à chacune de ses extrémités avec les ouvertures à des extrémités opposées de la chambre, chacune des deux ouvertures étant fermée par un couvercle (5), les couvercles (5) à des extrémités opposées de la chambre (4) s'ouvrant lorsqu'une pression est appliquée sur l'extérieur de la capsule (3).

2. Dispositif selon la revendication 1, comprenant une capsule (3) ayant au moins deux chambres (4), chacune étant remplie d'un ingrédient différent (2), de préférence au moins un ingrédient (2) étant de la nicotine ou un dérivé de nicotine.

3. Dispositif selon la revendication 1 ou 2, dans lequel un couvercle (5) ferme plus d'une ouverture à chaque extrémité ou les couvercles (5) à une extrémité consistent en au moins deux couvercles interconnectés (5).

4. Dispositif selon une ou plusieurs des revendications précédentes, dans lequel l'au moins une chambre (4) a une forme tubulaire et la capsule (3) et la chambre (4) sont souples lorsqu'une pression est appliquée depuis l'extérieur.

5. Dispositif selon une ou plusieurs des revendications précédentes, dans lequel les ingrédients (2) sont choisis parmi un ou plusieurs éléments parmi le groupe constitué de la nicotine, de dérivés de nicotine, d'agents chimiques de génération de vapeur, d'agents chimiques de génération de chaleur, et d'arômes.

6. Dispositif selon une ou plusieurs des revendications précédentes, dans lequel les ingrédients (2) sont à l'état liquide ou dissous, de préférence une solution d'un ou plusieurs ingrédients dissous dans un solvant.

7. Dispositif selon une ou plusieurs des revendications précédentes, dans lequel les couvercles (5) sont fixés et maintenus sur la capsule (3) uniquement sur la base d'un ajustement à frottement, en particulier un ajustement de la circonférence externe du couvercle contre la circonférence interne de la chambre.

8. Dispositif selon une ou plusieurs des revendications précédentes, dans lequel le couvercle (5) a une circonférence externe légèrement plus grande que la circonférence interne de la chambre (4) à l'extrémité où se trouve l'ouverture.

9. Dispositif selon une ou plusieurs des revendications précédentes, dans lequel les couvercles (5) ont un type de forme tronconique avec un diamètre inférieur à l'extrémité de fond (9) du couvercle et un diamètre supérieur à l'extrémité de tête (11), ou la circonférence de fond du couvercle est plus petite que la circonférence de tête.

10. Dispositif selon une ou plusieurs des revendications précédentes, dans lequel la ou les chambres (4) ont une forme tubulaire, la chambre (4), de préférence, s'effilant avec une augmentation de circonférence vers chaque extrémité et une circonférence plus petite au milieu.

11. Dispositif selon une ou plusieurs des revendications précédentes, dans lequel la capsule et les chambres sont faites d'un matériau polymère qui est élastique, de telle sorte que les parois de chambre sont élastiquement compressibles par un impact de doigts d'un utilisateur sur la capsule.

12. Dispositif selon une ou plusieurs des revendications précédentes, le dispositif comprenant un filtre côté bouche (21), un corps d'entrée (23) et un support de capsule (22) entre le filtre côté bouche (21) et le corps d'entrée (23), le support de capsule (22) ayant une paroi entourant une cavité et la cavité comprenant la capsule (3).

13. Dispositif selon la revendication 12, le dispositif comprenant un matériau absorbant à l'intérieur de la cavité, de préférence le matériau absorbant absorbe l'ingrédient et l'ingrédient se déplace à travers la paroi de la cavité afin d'être emporté par l'air s'écoulant au niveau des surfaces externes.

14. Dispositif selon une ou plusieurs des revendications précédentes, dans lequel la cavité comprend des espaces vides (18) à chaque extrémité de la capsule, ayant une taille qui permet aux couvercles de s'éloigner de la chambre lorsqu'ils sont retirés de la chambre.

15. Dispositif selon une ou plusieurs des revendications précédentes, dans lequel une surenveloppe (19) est prévue, recouvrant au moins le corps d'entrée (23) et le support de capsule (22) et, facultativement, également le filtre côté bouche (21).
